# EUROPEAN PATENT APPLICATION

(11) **EP 2 028 265 A1**
(43) Date of publication of application: **25.02.2009**
(21) Application number: 07114696.3
(22) Date of filing: 21.08.2007
(51) Int. Cl.: C12N 1/04, C12N 1/18

(54) **Compositions for the release and protection of instant active dry yeasts**

(71) Applicant: Beldem, 5300 Andenne (BE)
(72) Inventor: De Pauw, Paul, B-4219, WASSEIGES (BE); El Mejdoub, Thami, B-5030, GEMBLOUX (BE); Thonart, Philippe, B-5081, LA BRUYERE (BE)
(74) Representative: pronovem

(57) **Abstract**

The present invention provides a composition for preserving the stability, the fermentative activity and for favouring the release of instant active dry yeasts.

A composition of the invention can be in liquid, pasta-like or powdered form.

The invention concerns also the method for preparing said composition and its different applications.

## Description

### Field of the invention

The present invention provides a composition for preserving the stability, the fermentative activity and for favouring the release of instant active dry yeasts.

A composition of the invention can be in liquid, pasta-like or powdered form.

The invention concerns also the method for preparing said composition and its different applications.

### Background

Most living organisms are sensitive to their surrounding environment. Exposure of yeasts or lactobacilli to moisture or air destabilizes them rather quickly even when dried and certainly if not kept refrigerated.

Producers of powdered bakery mixes experience the limitations of including yeast in their mixes.

Quite rapidly, non-protected yeasts, i.e. in contact with different harmful factors, start to degrade, losing their fermentative power. As a consequence, when the packed mix is used by the consumer, the level of yeast activity is insufficient to produce the carbon dioxide required to fully expand the cell structure of the dough. Thus, since the dough does not adequately rise, the resulting baked product is of poor quality for the consumer.

One approach is to pack yeast separately in smaller bags under vacuum or conditioned air like CO₂, nitrogen or argon and seal them without leakages. But this necessitates that the yeast is separated from the remaining components of the dry mix, liquid mix or pasta like mix or that the mix is packed together with the yeast under vacuum or conditioned air and sealed without leakages.

Furthermore, because of economic and technical reasons, the packaging weight is limited to max 10 kg or more often to 0.5 kg. This is an expensive alternative due to the high packaging costs for small amounts of yeast or yeast-containing mixes per package and the use of high technological packaging machines.

Another method to increase the protection of active dry micro-organisms is the encapsulation. US Patent 6,261,613 discloses the encapsulation of particles, such as yeast particles, in a fat in a beta or beta prime form. The coating material can further contain emulsifiers such as those found in hydrogenated vegetable oil. However, the coating allows the release of the yeast only in a limited temperature range of about 40°C to about 55°C. Those temperatures cause partial inactivation of the yeast. Moreover only part of the yeast is released.

Encapsulation is a quite expensive process as it requires the use of specific equipment and the input of high amounts of energy.

In the FR 2 233 941, a composition made of active dry yeasts and oil is described for enhancing the stability of the yeasts. It is explained that this combination confers a stability that allows the further addition of flour. Indeed, like in US 2,523,483, each yeast granule must be completely covered with the oil before adding the flour.

### Summary of the invention

The present invention provides a composition for preserving yeasts fermentative activity, comprising (or consisting of):
- instant active dry yeast particles, and
- at least one biocompatible oil and/or at least one biocompatible paraffin, wherein the weight ratio of said oil(s) and/or paraffin(s) to said instant dry active yeasts is higher than 0.4:1, preferably higher than 0.6:1, and even more preferably higher than 0.7:1,
wherein the dry matter content (DMC) of the composition is higher than (about) 85 wt% and lower than (about) 98 wt%, preferably higher than (about) 87 wt% and lower than (about) 98 wt%, preferably comprised between (about) 89 wt% and (about) 98 wt%, more preferably comprised between (about) 90 wt% and (about) 98 wt%, more preferably comprised between (about) 92 wt% and (about) 98 wt%, more preferably comprised between (about) 92 wt% and (about) 97 wt%, more preferably comprised between (about) 92 wt% and (about) 96 wt%, more preferably comprised between (about) 93 wt% and (about) 95 wt%, even more preferably comprised between (about) 94 wt% and (about) 95 wt%, and
wherein all components are homogenously dispersed.

A composition of the invention, for preserving yeasts fermentative activity, can further comprise at least one hydrophilic component.

More particularly, a composition of the invention, for preserving yeasts fermentative activity, can comprise (or consist of):
- instant active dry yeast particles,
- at least one biocompatible oil and/or at least one biocompatible paraffin, wherein the weight ratio of said oil(s) and/or paraffin(s) to said instant active dry yeasts is higher than 0.4:1, preferably higher than 0.6:1, and even more preferably higher than 0.7:1, and
- at least one biocompatible hydrophilic component,
wherein the DMC of the composition is higher than (about) 85 wt% and lower than (about) 98 wt%, preferably higher than (about) 87 wt% and lower than (about) 98 wt%, preferably comprised between (about) 89 wt% and (about) 98 wt%, more preferably comprised between (about) 90 wt% and (about) 98 wt%, more preferably comprised between (about) 92 wt% and (about) 98 wt%, more preferably comprised between (about) 92 wt% and (about) 97 wt%, more preferably comprised between (about) 92 wt% and (about) 96 wt%, more preferably comprised between (about) 93 wt% and (about) 95 wt%, even more preferably comprised between (about) 94 wt% and (about) 95 wt%, and wherein all components are homogenously dispersed.

Preferably, in a composition of the invention, the dry matter content of the mixture consisting of said instant active dry yeast particles and said biocompatible hydrophilic component(s) is higher than (about) 85 wt% and lower than (about) 98 wt%, preferably higher than (about) 87 wt% and lower than (about) 96 wt%, preferably comprised between (about) 88 wt% and (about) 96 wt%, preferably comprised between (about) 89 wt% and (about) 96 wt%, more preferably comprised between (about) 89 wt% and (about) 95 wt%, even more preferably comprised between (about) 89 wt% and (about) 94 wt%.

Preferably, in a composition of the invention, said oil(s) consists of (water-free) edible oil(s).

The oil(s) can be selected from the group consisting of corn oil, peanut oil, coco oil, cocoa oil, olive oil, rapeseed oil, canola oil, nut oil, soybean oil, walnut oil, palm oil, palm Kernel oil, cottonseed oil, linseed oil, rice bran oil, safflower oil, sesame oil and sunflower oil.

Preferably, said oil is rapeseed oil. More preferably, said oil is sunflower oil.

More particularly, said oil(s) has/have a poly-unsaturated fatty acids content of at least 15 wt%, preferably of at least 20 wt% or 30 wt%, more preferably of at least 40 wt% or 50 wt%, or even of at least 60 wt% or 70 wt%, based on the total fatty acid weight of said oil.

And more preferably, said oil(s) has/have a linoleic acid content of at least 9 wt%, preferably of at least 15 wt%, preferably of at least 20 wt% or 30 wt%, more preferably of at least 40 wt% or 50 wt%, or even more preferably of at least 60 wt% or 70 wt%, based on the total fatty acid weight of said oil.

Preferably, said paraffin(s) consist(s) of (water-free) edible paraffin(s). And more particularly, said paraffin(s) has/have a kinetic viscosity comprised between 14 and 200 mm²/sec, preferably between 50 and 80 mm²/sec. Said paraffin(s) is/are liquid (i.e. pourable) at room temperature.

Preferably, in a composition of the invention, said hydrophilic component(s) exhibit(s) a dry matter content comprised between (about) 85 wt% and (about) 99 wt%, more preferably between (about) 86 wt% and (about) 96 wt%, more preferably between (about) 87 wt% and (about) 95 wt%, and even more preferably between (about) 88 wt% and (about) 94 wt%.

In a composition according to the invention, said hydrophilic component(s) can be selected from the group consisting of flours, emulsifiers, gums (such as gum arabic, alginate, carrageenan, caroube, guar, etc), pectin, gluten, yeast cell wall, glucans (such as, beta glucans, cellulose, starch, dextrins, maltodextrins, etc), yeast extract, yeast autolysates, modified starch, modified cellulose (such as carboxymethyl cellulose, etc) and edible (amorphous) silica.

Said emulsifier(s) can be sodium stearoyl lactylate, diacetyltartaric acid esters of mono- and diglycerides composed of fatty acids, hydrogenated or not, like stearate or palmitate or combinations thereof. Said emulsifier(s) can also be sucrose esters with different monoester content, potassium oleate, phospholipids, lecithins.

Said emulsifier(s) are preferably in the form of an aqueous solution.

Each of said emulsifiers or any combination of 2, 3 or more of said emulsifiers is envisaged in a composition of the invention.

Preferably, said hydrophilic component(s) is/are flour(s). Said flour can be from cereals, roots, beans and/or peas. Said flours can be a mixture of 2 or more different flours.

More preferably, said flour(s) is/are cereals flour(s), and more particularly is wheat flour.

Each of said flours or any combination of 2, 3 or more of said flours is envisaged in a composition of the invention.

In a composition according to the invention, said instant active dry yeasts can be baker's yeasts, oenological yeasts, brewery yeasts, ethanol producing yeasts, and/or probiotic yeasts.

Each of said yeasts or any combination of 2, 3 or more of said yeasts is envisaged in a composition of the invention.

Preferably, said instant active dry yeasts are baker's yeasts, more particularly are Saccharomyces yeast strains.

Preferably, said instant active dry yeasts have a dry matter content comprised between 90 wt% and 98 wt%, more preferably comprised between 92 wt% and 98 wt%, more preferably comprised between 90 wt% and 95 wt%, more preferably comprised between 92 wt% and 95 wt%, and even more preferably comprised between 92 wt% and 94 wt%.

A composition of the invention can further comprise at least one antioxidant. Said antioxidant(s) can be selected from the group consisting of alphatocopherol, butylated hydroxyanisole (BHA) and butylated hydroxytoluene (BHT).

Each of said antioxidants or any combination of 2, or 3 of said antioxidants is envisaged in a composition of the invention.

A composition of the invention can further comprise lactic acid bacteria, such as Lactobacilli, Lactococcus, Pediococci, Leuconostoc, Weissella and/or Bifidobacterium. Preferably, said lactic acid bacteria are selected from the group consisting of *Lactobacillus plantarum, Lactobacillus brevis, Lactobacillus acidophilus, Lactobacillus reuteri, Lactococcus lactis, Weissella confusa* and *Bifidobacterium longum.*

Each of said bacteria or any combination of 2, 3 or more of said bacteria is envisaged in a composition of the invention.

A composition according to the invention can be in a liquid (i.e. pourable), a paste or a powder form.

Preferably, a composition according to the invention is in a powder form.

The present invention also provides a method for preparing a composition of the invention.

The components (or ingredients) are those mentioned for, in the amounts specified for, a composition of the invention.

More particularly, a method of the invention comprises the steps of mixing, for an homogeneous distribution (or dispersion) of all components:
- instant active dry yeasts, in the form of particles, preferably with a dry matter content higher than 90 wt%, more preferably comprised between 90 wt% and 98 wt%, more preferably comprised between 92 wt% and 98 wt%, more preferably comprised between 90 wt% and 95 wt%, even more preferably comprised between 92 wt% and 95 wt%, and even more preferably comprised between 92 wt% and 94 wt%,
- at least one biocompatible oil and/or at least one biocompatible paraffin, wherein the weight ratio of said oils and/or paraffins to said instant active dry yeasts is higher than 0.4:1, preferably higher than 0.6:1, and even more preferably higher than 0.7:1, and
- optionally, at least one biocompatible hydrophilic component,
wherein all components are added in an amount such that the dry matter content of the composition is higher than (about) 85 wt% and lower than (about) 98 wt%, preferably higher than (about) 87 wt% and lower than (about) 98 wt%, preferably comprised between (about) 89 wt% and (about) 98 wt%, more preferably comprised between (about) 90 wt% and (about) 98 wt%, more preferably comprised between (about) 92 wt% and (about) 98 wt%, more preferably comprised between (about) 92 wt% and (about) 97 wt%, more preferably comprised between (about) 92 wt% and (about) 96 wt%, more preferably comprised between (about) 93 wt% and (about) 95 wt%, even more preferably comprised between (about) 94 wt% and (about) 95 wt%.

A method according to the present invention can be used for preserving yeasts fermentative activity.

More particularly, a method according to the present invention can be used for preserving yeasts fermentative activity and stability.

More preferably, a method according to the present invention can be used for preserving yeasts fermentative activity and stability, while favouring the release of the yeasts in the subsequent use steps, such as the steps for preparing a food product.

Preferably, in a method of the invention, said oil(s) consists of edible oil(s). Said oil(s) can be selected from the group consisting of corn, peanut, coco, cocoa, olive, rapeseed, canola, nut, soybean, walnut, palm, palm Kernel, cottonseed, linseed, rice bran, safflower, sesame, and sunflower oils.

More particularly, said oil(s) has/have a poly-unsaturated fatty acids content of at least 15 wt%, preferably of at least 20 wt% or 30 wt%, more preferably of at least 40 wt% or 50 wt%, or even of at least 60 wt% or 70 wt%, based on the total fatty acid weight of said oil.

And more preferably, said oil(s) has/have a linoleic acid content of at least 9 wt%, preferably of at least 15 wt%, preferably of at least 20 wt% or 30 wt%, more preferably of at least 40 wt% or 50 wt%, or even more preferably of at least 60 wt% or 70 wt%, based on the total fatty acid weight of said oil.

Preferably, the paraffin(s) used consist(s) of edible paraffin(s).

More particularly, said paraffin(s) has/have a kinetic viscosity comprised between 14 mm²/sec and 200 mm²/sec, preferably between 50 mm²/sec and 80 mm²/sec.

Said paraffin(s) can be a liquid paraffin obtained from Brenntag NV.

In a method of the invention, said hydrophilic component(s) preferably exhibit(s) a dry matter content comprised between (about) 85 wt% and (about) 99 wt%, more preferably between (about) 86 wt% and (about) 96 wt%, more preferably between (about) 87 wt% and (about) 95 wt%, and even more preferably between (about) 88 wt% and (about) 94 wt%.

In particular, said hydrophilic component(s) can be selected from the group consisting of flours, emulsifiers, gums (such as gum arabic, alginate, carrageenan, caroube, guar, etc), pectin, gluten, yeast cell wall, glucans (such as, beta glucans, cellulose, starch, dextrins, maltodextrins, etc), yeast extract, yeast autolysates, modified starch, modified cellulose (such as carboxymethyl cellulose, etc) and edible (amorphous) silica.

Said emulsifier(s) can be sodium stearoyl lactylate, diacetyltartaric acid esters of mono- and diglycerides composed of fatty acids, hydrogenated or not, like stearate or palmitate or combinations thereof. Said emulsifier(s) can also be sucrose esters with different monoester content, potassium oleate, phospholipids, lecithins.

Said emulsifier(s) are preferably in the form of an aqueous solution.

Each of said emulsifiers or any combination of 2, 3 or more of said emulsifiers is envisaged in a method of the invention.

Preferably, said hydrophilic component(s) is/are flour(s), more preferably is/are cereal flour(s).

Said instant active dry yeasts can be baker's yeasts, oenological yeasts, brewery yeasts, ethanol producing yeasts, and/or probiotic yeasts.

Preferably, said instant active dry yeasts are baker's yeasts, more particularly are Saccharomyces yeast strains.

A method of the invention can further comprise, before or after mixing the IADY, the oil(s) and/or paraffins and possibly (optionally) the hydrophilic component(s), the step of adding at least one antioxidant. Said antioxidant can be selected from the group consisting of alphatocopherol, butylated hydroxyanisole (BHA) and butylated hydroxytoluene (BHT).

A method of the invention can further comprise the step of adding lactic acid bacteria, such as lactobacilli. Said addition can be performed before or after mixing the IADY, the oil(s) and/or paraffins and possibly (optionally) the hydrophilic component(s).

Preferably, said lactic acid bacteria are Lactobacilli, Lactococcus, Pediococci, Leuconostoc, Weissella and/or Bifidobacterium strains. Preferably, said lactic acid bacteria are selected from the group consisting of *Lactobacillus plantarum, Lactobacilllus brevis, Lactobacillus acidophilus, Lactobacillus reuteri, Lactococcus* lactis, *Weissella confusa* and *Bifidobacterium longum.*

### Brief description of the figure

Figure 1 represents the balance unexpectedly found between the release of the yeast (expressed in terms of relative fermentative activity), the dry matter content and the stability of a composition according to the invention.

Figure 2, referring to example 6, shows the relation between release of yeast and the dry matter of the composition. A complete release was observed when the composition dry matter content is less than 95.89%.

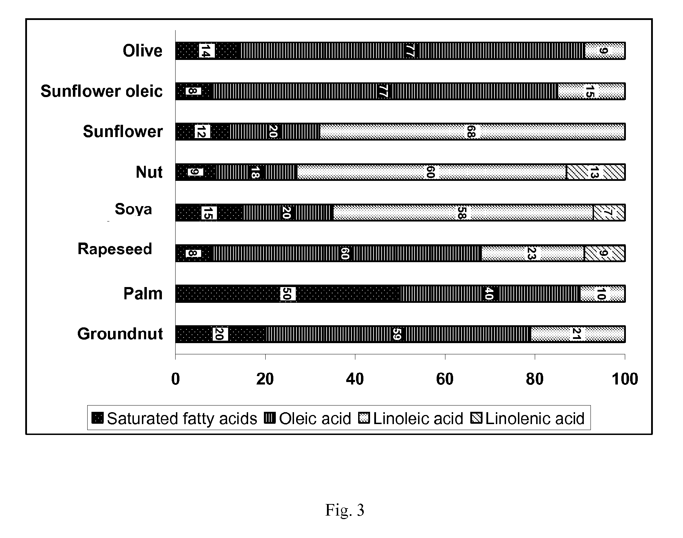

Figure 3 represents different oils that can be used in a composition and method of the invention, and their composition having regard to the saturated fatty acids content, oleic acid content, linoleic acid content and linolenic acid content, expressed as relative percentages to the total fatty acids content of different oils.

### Detailed description of the invention

The present invention relates to a non conventional approach for protecting instant active dry yeasts, even at room temperature (20°C), against oxidation or other instability provoking factors, while favouring their release in a subsequent use.

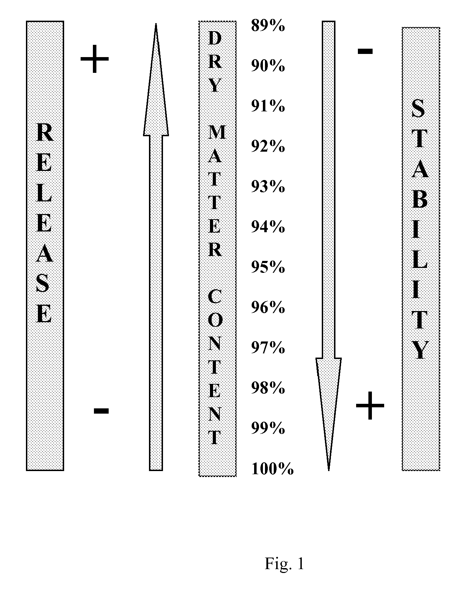

The present invention is based on the surprising discovery represented by fig. 1. Indeed, it was found an unexpected balance between the release of the yeasts (or fermentative activity), the dry matter content and the stability of a composition according to the invention.

In the context of the invention, the term "instant active dry yeast(s)" (also referred to as IADY) refers to yeast that has been submitted to drying processes employing mild conditions for preserving the yeast activity and that do not require re-hydration before use, contrary to active dry yeast (ADY), which needs to be re-hydrated before use.

Up to the present invention, it was thought that the drier the better having regard to the fermentative activity, and the trend was to develop products wherein the moisture of the IADY is lower than 5%. Indeed, contrary to the common general knowledge, in the context of the present invention, the contact of the yeasts with the hydrophilic component(s) does not affect the fermentative activity of the yeasts.

The present invention provides a composition comprising (or consisting of) instant active dry yeast particles, optionally partially rehydrated (in particular for meeting the DMC as specified for a composition according to the invention), and at least one biocompatible oil and/or at least one biocompatible paraffin, said instant active dry yeast particles being homogeneously dispersed in said biocompatible oil(s) and/or paraffin(s).

Preferably, a composition of the invention further comprises at least one biocompatible hydrophilic component, all components being homogeneously dispersed (or distributed).

More particularly, a composition of the invention comprises (or consists of):
- instant active dry yeast particles,
- at least one biocompatible oil and/or at least one biocompatible paraffin, and
- optionally, at least one biocompatible hydrophilic component,
wherein all components are homogeneously dispersed.

More particularly, a composition is provided, for preserving yeasts fermentative activity, comprising (or consisting of):
- instant active dry yeast particles,
- at least one biocompatible oil and/or at least one biocompatible paraffin, wherein the weight ratio of said oil(s) and/or paraffin(s) to said instant dry active yeasts is higher than 0.4:1, preferably higher than 0.6:1, and even more preferably higher than 0.7:1, and
- optionally, at least one biocompatible hydrophilic component, preferably in powder form,
wherein all components are added in an amount such that the dry matter content of the composition is higher than (about) 85 wt% and lower than (about) 98 wt%, preferably higher than (about) 87 wt% and lower than (about) 98 wt%, preferably comprised between (about) 89 wt% and (about) 98 wt%, more preferably comprised between (about) 90 wt% and (about) 98 wt%, more preferably comprised between (about) 92 wt% and (about) 98 wt%, more preferably comprised between (about) 92 wt% and (about) 97 wt%, more preferably comprised between (about) 92 wt% and (about) 96 wt%, more preferably comprised between (about) 93 wt% and (about) 95 wt%, even more preferably comprised between (about) 94 wt% and (about) 95 wt%, and
wherein all components are homogeneously dispersed.

Preferably, in a composition of the invention, the dry matter content of the mixture consisting of said instant active dry yeast particles and said biocompatible hydrophilic component(s) is higher than (about) 85 wt% and lower than (about) 98 wt%, preferably higher than (about) 87 wt% and lower than (about) 96 wt%, preferably comprised between (about) 88 wt% and (about) 96 wt%, preferably comprised between (about) 89 wt% and (about) 96 wt%, more preferably comprised between (about) 89 wt% and (about) 95 wt%, even more preferably comprised between (about) 89 wt% and (about) 94 wt%.

Preferably, in a composition of the invention said biocompatible hydrophilic component(s) is/are not optional.

As used in the context of the present invention, the term "about" means +/- 0.5%, unless the context clearly dictates otherwise. For example, "about 87%" is meant to include 86.5% and 87.5%, and any real number comprised between 86.5% and 87.5%.

In a composition of the invention, all species of yeasts are envisaged. Yeasts species with well known functionalities such as for example brewery yeast, baker's yeast - including sugar- and freeze tolerant and/or resistant yeast - and oenological yeasts, are preferred in the context of the invention.

In a preferred composition of the invention, the yeasts are baker's yeasts.

Yeasts with other functionalities, such as yeasts producing primary or secondary metabolites (ethanol, glutathione, glucans, etc.) or yeasts with probiotic activity or fodder yeast for food and feed applications, can also be used.

Besides the yeasts, further species of fungi, such as moulds, with all possible functionalities, can be a component of a composition according to the invention.

Besides the yeasts, a composition according to the invention can (further) comprise prokaryotic strains with all kind of functionalities such as, but not limited to, strains for production of primary metabolites (lactic acid, etc), strains for production of secondary metabolites (enzymes, antibiotics, etc), strains used to produce active or inactive sourdoughs or sourdough mixes, pro- and prebiotic strains.

More specifically, a composition of the invention can further comprise strains of lactic acid bacteria like *Lactobacillus, Leuconostoc, Weissella, Bifidobacterium* and *Pediococcus.*

Combinations of the above-mentioned strains and species are also envisaged in the context of the present invention.

Preferably, in a composition of the invention, said instant active dry yeast particles have a dry matter content comprised between 90 wt% and 98 wt%, more preferably comprised between 92 wt% and 98 wt%, more preferably comprised between 90 wt% and 95 wt%, more preferably comprised between 92 wt% and 95 wt%, and even more preferably comprised between 92 wt% and 94 wt%.

All components of a composition of the invention are biocompatible with the yeasts (i.e. they cause no harm to the yeasts).

Preferably, they are also suitable for animal and/or human consumption.

In the context of the present invention, the term "oil(s)" refers to an arbitrary group of certain common substances that are in a (unctuous, viscous) liquid state at ambient (ordinary) temperatures (more particularly at working or processing temperatures, preferably at temperatures higher than about 15°C) and that are hydrophobic, such as vegetable oils, animal fats, and essential oils.

One oil or a mixture of 2, 3 or more different oils can be used.

Said oil(s) are preferably edible oil(s).

Said edible oil(s) can be selected from the group consisting of corn, peanut, coco, cocoa, olive, rapeseed, canola, nut, soybean, walnut, palm, palm Kernel, cottonseed, linseed, rice bran, safflower, sesame, and sunflower oils.

Preferably, sunflower, nut, soybean, and/or rapeseed oil is/are used. More preferably rapeseed oil is used. Even more preferably sunflower oil is used.

It was observed that oil(s) having a high poly-unsaturated fatty acids content, confer(s) a better stability to a composition of the invention (the higher, the better).

An oil having a poly-unsaturated fatty acids content of at least 20 wt%, preferably of at least 30 wt%, 40 wt% or 50 wt%, or even of at least 60 wt% or 70 wt%, based on the total fatty acid weight of said oil, is particularly suitable.

And more preferably, said oil(s) has/have a linoleic acid content of at least 9 wt%, preferably of at least 20 wt% or 30 wt%, more preferably of at least 40 wt% or 50 wt%, or even of at least 60 wt% or 70 wt% based on the total fatty acid weight of said oil.

The paraffin(s) used consist(s) preferably of edible paraffin(s).

Said paraffin(s) has/have preferably a kinetic viscosity comprised between 14 and 200 mm²/sec, preferably between 50 and 80 mm²/sec.

The hydrophilic component(s) to be used in a composition of the invention preferably exhibit(s) a dry matter content comprised between (about) 85 wt% and (about) 99 wt%, more preferably between (about) 86 wt% and (about) 96 wt%, more preferably between (about) 87 wt% and (about) 95 wt%, and even more preferably between (about) 88 wt% and (about) 94 wt%.

Contrary to what was thought, the moisture introduced by said hydrophilic component(s), in contact with the IADY, does not affect the relative fermentative activity and/or improves the release of said IADY and still allows a good stability of the composition.

In the context of the present invention, the term "hydrophilic component(s)" refers to any component(s) that can transiently bind with water (H₂O) through hydrogen bonding and/or capillarity.

In a composition according to the invention, said hydrophilic component(s) can be selected from the group consisting of flours, emulsifiers, gums (such as gum arabic, alginate, carrageenan, caroube, guar, etc), pectin, gluten, yeast cell wall, glucans (such as, beta glucans, cellulose, starch, dextrins, maltodextrins, etc), yeast extract, yeast autolysates, modified starch, modified cellulose (such as carboxymethyl cellulose, etc) and edible (amorphous) silica.

A mixture of 2, 3 or more of said hydrophilic component(s) can be used in a composition of the invention.

Said hydrophilic component(s) can be one, two or more emulsifiers selected from the group consisting of sodium stearoyl lactylate, diacetyltartaric acid esters of mono- and diglycerides composed of fatty acids, hydrogenated or not, like stearate or palmitate, sucrose esters with different monoester content, potassium oleate, phospholipids, lecithins.

Preferably, said hydrophilic component(s) is/are flour(s), such as different cereal flours, bean flours, pea flours or root flours, more particularly wheat flour or manioc flour. A mixture of 2, 3 or more flours can be used. A preferred flour is wheat flour.

Antioxidant(s) can be added to a composition of the invention. Said antioxidant(s) can be selected from the group consisting of alphatocopherol, butylated hydroxyanisole (BHA) and butylated hydroxytoluene (BHT).

A composition according to the invention can be in a liquid (i.e. pourable), a paste or a powder form.

A first preferred composition according to the invention comprises (or consists of):
- instant active dry yeast particles, and
- at least one biocompatible oil, wherein the weight ratio of said oil(s) to said instant dry active yeasts is higher than 0.4:1,
wherein the dry matter content of the composition is comprised between (about) 92 wt% and (about) 98 wt%, more preferably comprised between (about) 92 wt% and (about) 97 wt%, more preferably comprised between (about) 92 wt% and (about) 96 wt%.

In said first preferred composition of the invention, said IADY have a dry matter content comprised between 92 wt% and 95 wt%, and even more preferably comprised between 92 wt% and 94 wt%.

An example of a composition of the invention comprises:
- instant active dry yeast particles having a DMC of 95 wt%,
- at least one biocompatible oil, such as rapeseed oil and/or sunflower oil, wherein the weight ratio of said oil(s) to said instant dry active yeasts is 2:1, and
wherein the dry matter content of the composition is (about) 98 wt%.

A second preferred composition according to the invention comprises (or consists of):
- instant active dry yeast particles,
- at least one biocompatible oil, wherein the weight ratio of said oil(s) to said instant dry active yeasts is higher than 0.4:1, preferably higher than 0.7:1, and
- at least one biocompatible hydrophilic component, preferably in powder form,
wherein all components are added in an amount such that the dry matter content of the composition is comprised between (about) 92 wt% and (about) 98 wt%, more preferably comprised between (about) 92 wt% and (about) 97 wt%, more preferably comprised between (about) 92 wt% and (about) 96 wt%, and
wherein all components are homogeneously dispersed.

Preferably, in said second preferred composition of the invention, said hydrophilic component(s) is/are flour(s), more particularly, cereal flour(s), and even more particularly wheat flour.

Said flour(s) preferably exhibit(s) a dry matter content comprised between (about) 86 wt% and (about) 96 wt%, more preferably between (about) 87 wt% and (about) 95 wt%, and even more preferably between (about) 88 wt% and (about) 94 wt%.

Preferably, in said second preferred composition of the invention, said IADY have a dry matter content comprised between 92 wt% and 98 wt%, more preferably comprised between 92 wt% and 97 wt%, and even more preferably comprised between 95 wt% and 97 wt%.

A third preferred composition of the invention comprises:
- instant active dry yeast particles having a DMC comprised between 92 wt% and 98 wt%, more particularly comprised between 95 wt% and 98 wt%,
- at least one biocompatible oil, such as rapeseed oil and/or sunflower oil, wherein the weight ratio of said oil(s) to said instant dry active yeasts is higher than 0.7:1, more particularly is (about) 0.75:1, and
- flour, preferably cereal flour, more particularly wheat flour, having a DMC comprised between (about) 88 wt% and 94 wt%, more particularly of (about) 89 wt%,
wherein the dry matter content of the composition is comprised between (about) 92 wt% and (about) 95 wt%, and
wherein all components are homogeneously dispersed.

The different preferred compositions of the invention can further comprise the ingredients as specified herein.

The present invention also provides a method for preparing a composition of the invention.

A method of the invention can comprise the step of mixing the different components described for a composition according to the invention, for obtaining a homogeneous distribution of said different components throughout said composition.

More particularly, a method of the invention comprises the steps of mixing, for an homogeneous distribution of all components:
- instant active dry yeasts, in the form of particles,
- at least one biocompatible oil and/or at least one biocompatible paraffin, wherein the weight ratio of said oils and/or paraffins to said instant active dry yeasts is higher than 0.4:1, preferably higher than 0.6:1, and even more preferably higher than 0.7:1, and
- optionally, at least one biocompatible hydrophilic component,
wherein all components are added in an amount such that the dry matter content of the composition is higher than (about) 85 wt% and lower than (about) 98 wt%, preferably higher than (about) 87 wt% and lower than (about) 98 wt%, preferably comprised between (about) 89 wt% and (about) 98 wt%, more preferably comprised between (about) 90 wt% and (about) 98 wt%, more preferably comprised between (about) 92 wt% and (about) 98 wt%, more preferably comprised between (about) 92 wt% and (about) 97 wt%, more preferably comprised between (about) 92 wt% and (about) 96 wt%, more preferably comprised between (about) 93 wt% and (about) 95 wt%, even more preferably comprised between (about) 94 wt% and (about) 95 wt%.

Preferably, in a method of the invention, said instant active dry yeast particles have a dry matter content comprised between (about) 90 wt% and (about) 98 wt%, more preferably comprised between (about) 92 wt% and (about) 98 wt%, more preferably comprised between (about) 90 wt% and (about) 95 wt%, more preferably comprised between (about) 92 wt% and (about) 95 wt%, and even more preferably comprised between (about) 92 wt% and (about) 94 wt%.

IADY having a dry matter content higher than 95 wt% can be partially rehydrated to reduce their dry matter content, in particular to reduce their dry matter content down to 94 wt%, 93 wt%, 92 wt%, 91 wt% or 90 wt%. Any percentage (real number) between 94 wt% and 90 wt% is envisaged in a method of the invention.

Alternatively, IADY having a dry matter content comprised between 90 wt% and 98 wt% can result directly from the process used for their preparation.

A method according to the invention confers protection to the IADY against different instability provoking factors that cause loss of fermentative power.

In particular, this protection is conferred by the use of oil(s) and/or paraffin(s) combined with at least said one hydrophilic component.

The protection is further increased with the use of oil(s) exhibiting a poly-unsaturated fatty acids content higher than 20 wt% (based on the total weight of the oil).

More specifically, oil(s) having a high concentration of C18-2 cis polyunsaturated fatty acids (linoleic acid) give very good results in that regards.

Such oil(s) can be for example rapeseed oil, nut oil, soybean oil, sunflower oil or mixture thereof. Moreover, modified oil(s) are (commercially) available with the required level of poly-unsaturated fatty acids and more specifically of linoleic acid.

The physical properties of the paraffin(s) used also influence the degree of protection and thus of activity losses, in particular its/their viscosity. Especially, paraffin(s) with a viscosity comprised between 14 mm²/sec and 200 mm²/sec, or more specifically between 50 mm²/sec and 80 mm²/sec, show(s) good protecting ability.

The use of hydrophilic component(s) substantially improves the release (in term of relative fermentative activity) of the yeasts during its subsequent use. It is assumed that there is an exchange of water between the yeast and the hydrophilic component(s), which is not prejudicial to the yeast fermentative activity and which favours the release of the yeast in a subsequent use.

The present invention will be further demonstrated by the following examples. It should be noted that the present invention is by no means limited to these examples.

### EXAMPLES

### Example 1: accelerated test.

The method used in the examples to evaluate the influence of temperature and oxygen on the stability of the yeasts and yeast compositions is called "the accelerated test".

The various formulations or yeasts are treated as follow. They are incubated in vials at 55°C in presence or not of oxygen during 18 hours. The fermentative capacity of the treated yeast or yeast compositions were measured by the method described hereafter. The results are then compared to the fermentative capacity of untreated reference yeast.

Different doughs were prepared with the ingredients as listed in table 1.

The ingredients were mixed for 1 min at low speed and 5 min at high speed in a dough mixer (Kenwood Major).

After kneading, 10 g dough samples were placed in closed 20 ml vials. The vials were hermetically closed by a cap provided with a septum and incubated at 30°C.

The fermentative capacity (production of CO₂) was measured by a pressure gauge through a needle piercing the septum after 90 minutes.

The relative fermentative capacity of the accelerated test is expressed as the percentage of produced gas of each tested dough in comparison with the dough made with the untreated yeast of reference.

**Table 1**

| **Ingredient** | **Quantity** |
|---|---|
| Wheat flour (DUO - Ceres, Belgium) | 100g |
| Water | 55g |
| IADY, or IADY compositions | 1.5g, or 1.5g IADY equivalent* |

| | |
|---|---|
| * i.e. amount sufficient for obtaining the equivalent of 1.5g IADY. | |

### Example 2: baking test.

Another method used to measure the relative fermentative capacity is the baking test and is described below.

The relative fermentative capacity is the ratio (expressed as percentage) of bread volume obtained with the formulated yeast compared to the one obtained with the reference yeast used for the test.

The ingredients of the baking test are listed in table 2.

**Table 2**

| **Ingredient** | **Quantity** |
|---|---|
| Wheat flour (DUO - Ceres, Belgium) | 2000g |
| Water | 1180g |
| IADY | 30g |
| Salt | 40g |
| S-500 Controller(Puratos, Belgium) | 2% |

The ingredients (table 2) were mixed for 1.5 min at low speed and 6 min at high speed in a dough mixer (Prat). Temperature in the bakery was 25°C.

Dough temperature was 26°C.

After a bulk fermentation for 30 min the dough was divided in 600 g pieces and submitted to an intermediate proofing step of 20 min at 25°C.

A final proofing step was performed in a Koma fermentation room (60 min, 35°C, 95% relative humidity) before baking at 230°C for 35 min with steam in a Miwe Roll Inn oven.

After baking, the volume of 4 breads was evaluated by the rape seeds displacement method and the mean value of the volume of the breads made with a composition according to the present invention was compared to the value obtained for the breads made with the reference yeast.

### Example 3: Effect of yeast dry matter content on release.

In order to determine the effect of water on the yeast release from a composition of the invention consisting of IADY and oil, extruded fresh baker's yeast *(Saccharomyces cerevisiae)* particles were dried at different levels by fluidization.

The dry matter content of the IADY for each drying level is illustrated in the table 3.

**Table 3**

| **Yeast** | **DMC (wt%)** |
|---|---|
| Y1 | 88 |
| Y2 | 90 |
| Y3 | 92 |
| Y4 | 94 |
| Y5 | 96 |

Different compositions according to the invention, prepared by mixing IADY with different dry matter contents and sunflower oil, are illustrated in table 4. For each composition, 1 g IADY at different DMC was added to 0.75 g oil.

**Table 4**

| | **used quantity (w/w)** | | **DMC (wt%)** |
|---|---|---|---|
| | **Yeast** | **oil** | |
| **FY1** | 1 (Y1) | 0.75 | 94.46 |
| **FY2** | 1 (Y2) | 0.75 | 94.73 |
| **FY3** | 1 (Y3) | 0.75 | 96.89 |
| **FY4** | 1 (Y4) | 0.75 | 97.49 |
| **FY5** | 1 (Y5) | 0.75 | 99.00 |

The relative fermentative capacity was measured with the method as described in example 2. The results are presented in table 5.

**Table 5**

| **Formulations** | **Fermentative capacity (%)** |
|---|---|
| **FY1** | 100 |
| **FY2** | 100 |
| **FY3** | 100 |
| **FY4** | 80 |
| **FY5** | 72 |

It could be observed that the drying of yeast at DMC ranging between 88 wt% and 92 wt% enhances the further release of the yeast.

A very similar effect is obtained when wheat flour at 89% DMC is incorporated within the different compositions.

When the DMC of yeast exceeds 94%, the fermentative capacity falls down to 80% (and to almost 70% for a DMC of 96%).

These results, with those of the further examples, show that the hydrophilic components as used are a source of water for the IADY which improves the further release of the yeast.

### Example 4: Different compositions according to the invention.

Different preferred formulations of a composition according to the invention (oil, IADY and flour) have been prepared and are detailed in tables 6 and 7.

The Flour used with trademark "DUO Cereclass" is a conventional wheat flour from Ceres SA, Belgium

The IADY used is the standard IADY from Beldem SA for lean dough.

The oil used is a refined sunflower oil (from Cargill).

For the formulations in paste form (table 6), the IADY, oil and flour were added in a ratio (w:w:w) of 1:0.75:1.25 respectively.

For the formulations in powder form (table 7), the IADY, oil and flour were added in a ratio (w:w:w) of 1:0.75:3.25 respectively.

**Table 6**

| **DMC of the flour** (wt%) | **DMC of composition** (wt%) | **DMC of the mixture IADY/flour**(wt%) |
|---|---|---|
| 89 | 94.00 | 92.10 |
| 90 | 94.50 | 92.70 |
| 91 | 94.90 | 93.20 |
| 92 | 95.30 | 93.80 |
| 93 | 95.75 | 94.30 |
| 94 | 96.16 | 94.90 |
| 95 | 96.58 | 95.40 |
| 96 | 97.00 | 96.00 |

**Table 7**

| **DMC of the flour (wt%)** | **DMC of composition (wt%)** | **DMC of the mixture IADY/flour** (wt%) |
|---|---|---|
| 89 | 92.05 | 90.60 |
| 90 | 92.70 | 91.40 |
| 91 | 93.35 | 92.20 |
| 92 | 94.00 | 92.90 |
| 93 | 94.65 | 93.70 |
| 94 | 95.30 | 94.50 |
| 95 | 95.95 | 95.20 |
| 96 | 96.60 | 96,00 |
| 97 | 97.25 | 96.80 |

### Example 5: Effect of oil on the stability of yeast fermentative capacity in bakery mixes.

A yeast protected formulation was prepared by mixing the ingredients as in table 8 to obtain a complete bakery improver (also referred to as complete mix).

**Table 8**

| **Ingredient** | **Content** (wt%) |
|---|---|
| Refined palm | 20% |
| rapeseed oil | 16.25% |
| Emulsifier E472e (Puratos NV, Belgium) | 0.8% |
| Emulsifier E471 (Puratos NV, Belgium) | 2.35% |
| Sugars | 10.6% |
| IADY (Beldem SA, Belgium) | 25% |
| Norma (Beldem SA, Belgium) | 25% |

Norma is an inactive dry sourdough based on wheat flour.

The DMC of the mixture consisting of E472e, E471, sugars, IADY and Norma is 94.51 wt%.

The stability of the complete mix was evaluated by the Risograph method at different intervals during 4 months.

Dough was prepared by mixing the ingredients of table 9 for 2 min at low speed and 6 min at high speed (Diosna mixer).

**Table 9**

| **Ingredient** | **Quantity** |
|---|---|
| Wheat flour DUO(Ceres NV, Belgium) | 1500 g |
| Water | 855 g |
| Salt | 30 g |
| Sugar | 150 g |
| Complete mix | 300 g |

The temperature of the dough was about 26.5°C.

100 g of dough were put in the Risograph (National Manufacturing Inc., USA) equilibrated at 30°C (waterbath).

After 1 h the relative fermentative activity was measured based on pressure measured by a pressure probe integrated in the Risograph unit and recalculated in function of the value obtained with instant active dry baker's yeast (Beldem SA, Belgium).

Results are expressed as relative fermentative capacity and shown in table 10 (stability of IADY in complete mixes).

The non-protected reference is the instant active dry baker's yeast stored in an open package at room temperature (20°C).

**Table 10**

| | **Relative fermentative capacity (%)** | | | | |
|---|---|---|---|---|---|
| | **start** | **6 weeks** | **8 weeks** | **12 weeks** | **16 weeks** |
| **non- protected reference** | 100 | 85 | 78 | 72 | 65 |
| **Complete mix** | 100 | 82 | 78 | 81 | 82 |

The yeast part of the complete mix remains stable.

### Example 6.

The relative fermentative capacity as an indicator of the stability of the mix has been determined for different formulations of a composition according to the invention by the accelerated test (example 1). The results are shown in table 12.

The formulations of this example are in paste form. IADY, oil and flour were added in a ratio (w:w:w) of 1:0.75:1.25 respectively (table 11).

The flour used with trademark "DUO Cereclass" is a conventional wheat flour from Ceres SA, Belgium

The IADY used is the standard IADY from Beldem SA for lean dough

The oil used is refined sunflower oil (from Cargill).

**Table 11**

| **Batch n°** | **DMC of flour (%)** | **DMC of composition (wt%)** | **DMC of mixture IADY/flour** (wt%) |
|---|---|---|---|
| B191 | 89.00 | 94.35 | 92.47 |
| B192 | 90.15 | 94.63 | 92.84 |
| B193 | 91.11 | 95.07 | 93.43 |
| B194 | 92.20 | 95.46 | 93.95 |
| B195 | 93.18 | 95.89 | 94.52 |
| B196 | 93.90 | 96.03 | 94.71 |
| B197 | 95.37 | 96.30 | 95.07 |
| B198 | 96.12 | 96.57 | 95.43 |

**Table 12**

| **Batch** n° | **Relative fermentative capacity (%)** | |
|---|---|---|
| | *Effect of T° (55°C)* | *Effect of T° (55°C) and O₂* |
| B191 | 38 | 32 |
| B192 | 49 | 35 |
| B193 | 55 | 43 |
| B194 | 65 | 53 |
| B195 | 70 | 59 |
| B196 | 76 | 59 |
| B197 | 81 | 59 |
| B198 | 81 | 64 |

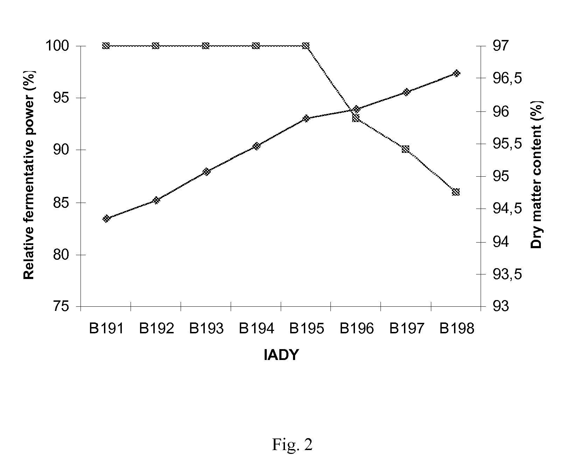

These results show the relation between the stability and the DMC of the composition. On the other hand Figure 2 demonstrates a complete release when the DMC of the composition is less than 95.89 wt%. These results are also illustrated by figure 1.

### Example 7.

Different formulations prepared with IADY (*S*. *cerevisiae*)*,* oil and different types of hydrophilic components, added in a ratio (w:w:w) of 1:0.75:0.075, were evaluated in terms of fermentative activity.

The DMC of the different compositions according to the invention are presented in table 13.

**Table 13**

| **Batch n°** | **hydrophilic components** | **DMC of composition** (wt%) | **DMC of mixture IADY/ hydrophilic components** (wt%) |
|---|---|---|---|
| **TO** | none | | |
| **T1** | Tween 80 | 97.8 | 96.3% |
| **T4** | Span 60 | 97.8 | 96.3% |
| **T7** | K Oléates | 94.5 | 92.4% |

The K-oleate used is an aqueous solution containing 20 wt% K-oleate (Christeyns NV).

The fermentative capacity was measured using the baking test as described in example 2.

The compositions according to the invention (IADY, oil and hydrophilic components) were added directly to the dough ingredients without pre-handling preparative step. Reference yeast is the instant active dry yeast (Beldem SA, Belgium) in the original vacuum packaging. The results are presented in table 14 (relative fermentative capacity of the formulation tested).

**Table 14**

| **Formulations** | **Relative fermentative capacity** (%) |
|---|---|
| **T0** | 67 |
| **T1** | 67 |
| **T4** | 66 |
| **T7** | 94 |

The results show that potassium oleate is effective for the release of yeast during kneading.

The relative fermentative capacity obtained for T1 and for T4 is similar to the one obtained for T0. Nevertheless, T1 and T4 formulations show a better stability than the reference.

### Example 8.

The effect of other hydrophilic compounds on the release of the protected yeast during kneading was evaluated. IADY, oil and hydrophilic compounds were added in a ratio (w:w:w) of 1:0.75:1.25 respectively. Table 15 shows the compounds tested together with the DMC.

**Table 15**

| **Formulations** | **hydrophilic components** | **DMC of the hydrophilic components (%)** | **DMC of composition** (wt%) | **DMC of mixture*** (wt%) |
|---|---|---|---|---|
| **F.F** | flour | 89 | 94.0 | 92.1 |
| **F.G** | Arabic gum | 91 | 94.9 | 93.2 |
| **F.M** | maltodextrin | 95 | 96.6 | 95.4 |

| | | | | |
|---|---|---|---|---|
| * mixture of IADY and hydrophilic component. | | | | |

The flour used is a wheat flour (Duo Cereclass, Ceres SA, Belgium). The arabic gum is obtained from Microbelcaps, Belgium. The maltodextrin is Glucidex^{®}, from Roquette, France.

The fermentative capacity of the various formulations was evaluated by a baking test (according to the method described in example 2).

Reference yeast is the instant active dry yeast (Beldem SA, Belgium) in the original vacuum packaging.

The formulated yeasts were added directly to the dough ingredients without pre-handling preparative step.

The results are shown in table 16.

**Table 16**

| **Formulations** | **Relative fermentative capacity** (%) |
|---|---|
| F.F | 100 |
| F.G | 87 |
| F.M | 79 |

The fermentative capacity of the formulation F.F (yeast protected with sunflower oil and flour) has been evaluated after storage for prolonged periods at room temperature.

The fermentative capacity was measured by the baking test (see example 2).

Reference yeast is the instant active dry yeast (Beldem SA, Belgium) in the original vacuum packaging.

Non-protected reference is the same yeast stored in an open package at room temperature (20°C).

The results are presented on table 17: Evolution of the relative fermentative capacity of formulation F.F during storage.

**Table 17**

| | **Relative fermentative capacity (%)** | | | |
|---|---|---|---|---|
| | **0 day** | **44 days** | **62 days** | **104 days** |
| **non-protected reference** | 100 | 81 | 78 | 68 |
| **F.F** | 100 | 90 | 89 | 88 |

The flour, exhibiting a DMC of 89 wt%, improves the release and the stability of the protected yeast.

### Example 9.

The formulations of this example are in paste form. IADY, oil and flour were added in a ratio (w:w:w) of 1:0.75:1.25 respectively.

The flour used (with trademark "DUO Cereclass") is a conventional wheat flour from Ceres SA, Belgium

The IADY used is the standard IADY from Beldem SA for lean dough

The oil used is refined sunflower oil (from Cargill).

Flour with a dry matter content of 89%, 96% and 100% were provided to prepare formulations of table 18. The samples of flour with a dry matter content of 96% and 100% were prepared by incubation for respectively 40 min and 24 hours in an oven at 105°C.

**Table 18**

| **Formulations** | **DMC of flour** | **DMC of composition** (wt%) | **DMC of mixture IADY/flou**r (wt%) |
|---|---|---|---|
| **F89** | 89% | 94.0 | 92.1 |
| **F96** | 96% | 97.0 | 96.0 |
| **F100** | 100% | 99.0 | 98.2 |

The degree of release and stability of the formulations were evaluated by baking tests (see the method of example 2).

For the degree of release, the formulated yeast was added directly to the dough ingredients without pre-handling preparative mixing step.

The results are presented in table 19.

**Table 19**

| **Formulations** | **Relative fermentative capacity** (%) |
|---|---|
| **F89** | 100 |
| **F96** | 76 |
| **F100** | 61 |

The release is lower when the flour is drier.

The stability of the yeast after 4 months at 20°C and without specific conditioning was measured by mixing the formulations in water at 37°C during 15 minutes before performing the baking method. The results are presented in table 20.

**Table 20**

| **Formulations** | **Relative fermentative capacity (%)** |
|---|---|
| **F89** | 88 |
| **F96** | 100 |
| **F100** | 100 |

Although the flour with dry substances higher than 96% did not allow a good release, the fermentative capacity was stable.

### Example 10.

Granules of yeast were coated with absorbent supports using fluidized bed technique.

The fluidized bed dryer used is an Aeromatic unit (NIRO AEROMATIC AG, VERFAHRENSTECHNISCHE, ANALGEN, BUBENDORF, Switzerland).

Various coating solutions with various hydrophilic supports were prepared (Table 21).

The liquid was sprayed through a nozzle into the fluidized bed dryer containing 500 grams of solid yeast particles. The solution was sprayed with an initial flow of 200 ml/h. The flow was then increased to reach 400 ml/h. The drying temperature was about 30°C.

The experiment was carried out to obtain an incorporation level as described in table 21.

Fermentative capacity of the samples was evaluated using the test described in example 1 with the exception that no treatment at 55°C was performed. The results are shown in table 21.

**Table 21.**

| **Batch** | **Type of coating compound** | **Repartition of coating compound(s) (w/w%)** | **Concentration coating before fluidisation (w%)** | **Composition after fluidisation (w coat/w yeast)** | **Relative fermentative capacity after 2 months (%)** |
|---|---|---|---|---|---|
| **B1** | Maltodextrin / Arabic gum | 80/20 | 50 | 40/100 | 83 |
| **B2** | Maltodextrin / Arabic gum | 80/20 | 50 | 80/100 | 82 |
| **B7** | Maltodextrin / Arabic gum | 80/20 | 50 | 50/100 | 80 |
| **B22** | Traviata R 80 | 100 | 20 | 34/100 | 84 |
| **B23** | Celyarom CW DD | 100 | 20 | 20/100 | 81 |
| **B24** | Arabic gum TAN 61 766 | 100 | 20 | 20/100 | 75 |
| **B25** | HP 20 | 100 | 20 | 20/100 | 82 |
| **B27** | MM 90 SA | 100 | 20 | 26/100 | 85 |

Arabic Gum (Acacia) purified and dried, from Microbelcaps, Belgium; maltodextrin (roferose dextrose monohydrate), Glucidex, from Roquette, France; Traviata R80 (a dried mild sour dough), and Celyarom CW DD (a dried yeast cell wall), from Beldem, Belgium; HP20 (E472e, a DATA-ester) and MM 90 SA (E471, a monoglyceride) from Puratos, Belgium.

No coating solution gave a protective effect to yeast during its storage at room temperature without specific conditioning. The range of relative fermentative capacity after 2 months without specific conditioning at 20°C is situated between 75% and 85%.

Observation by scanning electron microscopy of the coated yeast granules from batch B2 revealed cracks on the surface of the granules. These cracks eventually join the edges.

### Example 11: yeast stability in the presence of oil with different polyunsaturated fatty acids content.

Yeast formulations were prepared by mixing instant active dry yeast (Beldem SA, Belgium) with different types of oils according to table 22. The reference yeast used is the instant active dry yeast (Beldem SA, Belgium) conserved under vacuum.

The composition is made at a ratio of 0.75 g of oil for 1 g of yeast.

**Table 22.**

| **Formulations** | **Type of oil** | **Dilution factor of IADY in composition** | **DMC of yeast (wt%)** | **DMC of composition (wt%)** |
|---|---|---|---|---|
| **F1** | Rapeseed oil | 1.75 | 96.0% | 97.7% |
| **F3** | Sunflower oil | 1.75 | 96.0% | 97.7% |
| **F5** | High oleic sunflower oil | 1.75 | 96.0% | 97.7% |

The relative fermentative capacity has been evaluated according to the accelerated tests described in example 1.

The behaviour of the various yeast formulations in the accelerated test is presented in table 23.

**Table 23.**

| **Formulations** | **Relative fermentative capacity (%)** | |
|---|---|---|
| | **Effect T°** | **Effect T°/O2** |
| **Treated reference yeast** | 50 | 40 |
| **F1** | 68 | 66 |
| **F3** | 83 | 66 |
| **F5** | 65 | 71 |

The presence of oil in the protecting formulations allowed a resistance to inactivation with respect to the harmful effect of oxygen and temperature. Moreover refined sunflower oil (F3) with a higher content of unsaturated fatty acid shows a higher resistance than the others (F1, F5).

Similar results are obtained when other baker's yeast strain (Algist, Bruggeman, Belgium) or oenological or brewing yeast strains are tested in the same conditions.

### Example 12: Long-term stability of yeast fermentative capacity in the presence of oil and flour.

Yeast formulations were prepared by mixing instant active dry yeast (Beldem SA, Belgium) with different types of oils and with flour. The flour used (with trademark "DUO Cereclass") is a conventional wheat flour from Ceres SA, Belgium. The reference yeast used is the instant active dry yeast (Beldem SA, Belgium) conserved under vacuum.

The formulations of this example are in paste form. IADY, oil and flour were added in a ratio of 1:0.75:1.25 respectively (table 24). The flour fraction was added to the yeast-oil composition after 9 months of storage at room temperature.

**Table 24.**

| **Formulations** | **Type of oil** | **Dilution factor of IADY in composition** | **DMC of yeast (wt%)** | **DMC of composition (wt%)** |
|---|---|---|---|---|
| **F1** | Rapeseed oil | 3.0 | 96.0% | 93.8% |
| **F3** | Sunflower oil | 3.0 | 96.0% | 93.8% |
| **F5** | High oleic sunflower oil | 3.0 | 96.0% | 93.8% |

The degree of release was evaluated by adding the compositions according to the invention directly to the dough ingredients in the baking test without pre-handling preparative mixing step.

The fermentative capacity of the compositions has been evaluated by the baking test (see example 2) after storage for 10 months at room temperature. The results are presented in table 25.

**Table 25.**

| **Formulations** | **Relative fermentative capacity (%)after 10 months** |
|---|---|
| **Reference yeast under vacuum** | 100 |
| **Reference yeast not protected** | 50 |
| **F1** | 86 |
| **F3** | 92 |
| **F5** | 77 |

After 10 months of storage at room temperature and without any specific conditioning, the level of protection exerted by the rapeseed oil is about 86%, by the sunflower oil about 92% whereas the protection of the preparation yeast/oleic sunflower oil is only about 77%.

### Example 13: long-term stability of yeast fermentative capacity in the presence of paraffin oils.

Instant active dry yeast was mixed with hydrophobic compounds of various viscosities in the ratio 1/0.75 (w/w).

Two paraffin oils (Brenntag, Belgium), SIP MED 68 with a viscosity between 62 and 75 cSt (40°C) and SIP MED 15 with a viscosity between 14 and 16 cSt (40°C) were compared to olive oil.

The formulated yeasts as well as the non treated (non protected) reference yeast were stored at room temperature and evaluated in baking tests according to example 2 after 1, 2 and 3 months.

Reference yeast is the instant active dry yeast (Beldem SA, Belgium) in the original vacuum packaging.

Non protected reference is the same yeast stored in an open package at room temperature (20°C). The results are presented in table 26.

Formulated yeasts were mixed in water at 37°C during 15 minutes before handling. This treatment releases immediately the yeast and allows discriminating between the protection exerted by the paraffins and the release of the yeast.

**Table 26.**

| | **Relative fermentative capacity (%)** | | |
|---|---|---|---|
| | **1 month** | **2 months** | **3 months** |
| **non protected reference** | 81 | 43 | 28 |
| **Paraffin oil SIP MED 68 (°)** | 97 | 96 | 93 |
| **Paraffin oil SIP MED 15 (°)** | 87 | 88 | 85 |
| **Olive oil** | 99 | 97 | 93 |

| | | | |
|---|---|---|---|
| (°): Brenntag paraffin oil of respectively 69.8 cSt and 15 cSt (ASTM D445) | | | |

The presence of paraffin oils protects the yeast during storage and provides a good long-term stability of the yeast fermentative capacity in the absence of any specific physical storage conditions.

By adding a hydrophilic component with the DMC as specified for a composition of the invention, such as flour (e.g. with a DMC of about 89 wt%), more particularly such as wheat flour, the mixing step in water becomes unnecessary, said hydrophilic component, more particularly said (wheat) flour, favouring/promoting the release.

## Claims

1. A composition for preserving yeasts fermentative activity comprising:
- instant active dry yeast particles,
- at least one biocompatible oil and/or at least one biocompatible paraffin, wherein the weight ratio of said oil(s) and/or paraffin(s) to said instant dried active yeasts is higher than 0.4:1,
- optionally, at least one biocompatible hydrophilic component,
wherein the dry matter content of the composition is higher than 85 wt% and lower than 98 wt%, and wherein all components are homogenously dispersed.

2. A composition according to claim 1, wherein the dry matter content of the composition is higher than 90 wt% and lower than 98 wt%.

3. A composition according to claim 1, wherein the dry matter content of the composition is higher than 92 wt% and lower than 96 wt%.

4. A composition according to claim 1, wherein the dry matter content of the composition is higher than 92 wt% and lower than 95 wt%.

5. A composition according to any of claims 1 to 4, wherein said instant active dry yeast particles have a dry matter content comprised between 90 wt% and 98 wt%.

6. A composition according to any of claims 1 to 4, wherein said instant active dry yeast particles have a dry matter content comprised between 90 wt% and 95 wt%.

7. A composition according to any of claims 1 to 6, wherein said oil(s) consists of edible oil(s).

8. A composition according to any of claims 1 to 7, wherein said oil(s) is/are selected from the group consisting of corn, peanut, coco, cocoa, olive, rapeseed, canola, nut, soybean, walnut, palm, palm Kernel, cottonseed, linseed, rice bran, safflower, sesame and sunflower oils.

9. A composition according to any of claims 1 to 8, wherein said oil(s) has/have an unsaturated fatty acids content of at least 40 wt%, based on the total fatty acid weight of said oil.

10. A composition according to any of claims 1 to 9, wherein said oil(s) has/have a linoleic acid content of at least 9 wt%, based on the total fatty acid weight of said oil.

11. A composition according to any of claims 1 to 10, wherein said paraffin(s) consists of edible paraffin(s).

12. A composition according to any of claims 1 to 11, wherein said paraffin(s) has/have a kinetic viscosity comprised between 14 and 200 mm²/sec, preferably between 50 and 80 mm²/sec.

13. A composition according to any of claims 1 to 12, wherein said hydrophilic component(s) is/are selected from the group consisting of flours, emulsifiers, gums, pectin, gluten, glucans, yeast cell walls, yeast extract, yeast autolysates, modified starch, modified cellulose and edible amorphous silica.

14. A composition according to any of claims 1 to 12, wherein said hydrophilic component(s) is/are flour(s).

15. A composition according to claim 13 or 14, wherein said flour(s) is/are cereals flour(s).

16. A composition according to any of claims 13 or 15, wherein said emulsifier(s) is/are in the form of aqueous solution(s)*.*

17. A composition according to any of claims 1 to 16, wherein the mixture consisting of said instant active dry yeast particles and said hydrophilic component(s) has a dry matter content comprised between 85 wt% and 98 wt%.

18. A composition according to claim 17, wherein the dry matter content of said mixture is comprised between 89 wt% and 95 wt%.

19. A composition according to any of claims 1 to 18, further comprising at least one antioxidant selected from the group consisting of alphatocopherol, butylated hydroxyanisole (BHA) and butylated hydroxytoluene (BHT).

20. A composition according to any of claims 1 to 19, further comprising lactic acid bacteria, such as Lactobacilli, Lactococcus, Pediococci, Leuconostoc, Weissela and/or Bifidobacterium.

21. A composition according to claim 20, wherein said lactic acid bacteria are selected from the group consisting of *Lactobacillus plantarum, Lactobacillus brevis, Lactobacillus acidophilus, Lactobacillus reuteri, Lactococcus lactis, Weissela confusa* and *Bifidobacterium longum.*

22. A composition according to any of claims 1 to 21, wherein said instant active dry yeasts are baker's yeasts, including sugar- and freeze tolerant yeasts, oenological yeasts, brewery yeasts, ethanol producing yeasts, and/or probiotic yeasts.

23. A composition according to claim 22, wherein said yeasts are Saccharomyces yeast strains.

24. A composition according to any of claims 1 to 23 in a liquid, or paste or powder form.

25. A method for preserving yeasts fermentative activity comprising the steps of mixing, for an homogeneous distribution of all components:
- instant active dry yeasts,
- at least one biocompatible oil and/or at least one biocompatible paraffin, wherein the weight ratio of said oils and/or paraffins to said instant active dry yeasts is higher than 0,4:1, and
- optionally, at least one biocompatible hydrophilic component,
wherein all components are added in an amount such that the dry matter content of the composition is higher than 85 wt% and lower than 98 wt%.

26. A method according to claim 25, wherein the dry matter content of the composition is higher than 90 wt% and lower than 98 wt%.

27. A method according to claim 25, wherein the dry matter content of the composition is higher than 92 wt% and lower than 98 wt%.

28. A method according to claim 25, wherein the dry matter content of the composition is higher than 92 wt% and lower than 95 wt%.

29. A method according to any of claims 25 to 28, wherein said instant active dry yeast particles have a dry matter content comprised between 90 wt% and 98 wt%.

30. A method according to any of claims 25 to 28, wherein said instant active dry yeast particles have a dry matter content comprised between 90 wt% and 95 wt%.

31. A method according to any of claims 25 to 30, wherein said oil(s) consists of edible oil(s).

32. A method according to claim 31, wherein said oil(s) is/are selected from the group consisting of corn, peanut, coco, cocoa, olive, rapeseed, canola, nut, soybean, palm, walnut, palm Kernel, cottonseed, linseed, rice bran, safflower, sesame and sunflower oils.

33. A method according to any of claims 25 to 32, wherein said oil(s) has/have an unsaturated fatty acids content of at least 40 wt%, based on the total fatty acid weight of said oil.

34. A method according to any of claims 25 to 33, wherein said oil(s) has/have a linoleic acid content of at least 9 wt%, based on the total fatty acid weight of said oil.

35. A method according to any of claims 25 to 34, wherein said paraffin(s) consists of edible paraffin(s).

36. A method according to any of claims 25 to 35, wherein said paraffin(s) has/have a kinetic viscosity comprised between 14 and 200 mm²/sec, preferably between 50 and 80 mm²/sec.

37. A method according to any of claims 25 to 36, wherein said hydrophilic component(s) is/are selected from the group consisting of flours, emulsifiers, gums, pectin, gluten, glucans, yeast cell walls, yeast extract, yeast autolysates, modified starch, modified cellulose and edible amorphous silica.

38. A method according to any of claims 25 to 36, wherein said hydrophilic component(s) is/are flour(s).

39. A method according to claim 37 or 38, wherein said flour(s) is/are cereal flour(s).

40. A method according to claim 37 or 39, wherein said emulsifier(s) is/are in the form of aqueous solution(s).

41. A method according to any of claims 25 to 40, wherein the mixture consisting of said instant active dry yeast particles and said hydrophilic component(s) has a dry matter content comprised between 85 wt% and 98 wt%.

42. A composition according to claim 41, wherein the dry matter content of said mixture is comprised between 89 wt% and 95 wt%.

43. A method according to any of claims 25 to 42, further comprising at least one antioxidant selected from the group consisting of alphatocopherol, butylated hydroxyanisole (BHA) and butylated hydroxytoluene (BHT).

44. A method according to any of claims 25 to 43, further comprising lactic acid bacteria, such as Lactobacilli, Lactococcus, Pediococci, Leuconostoc, Weissella and/or Bifidobacterium.

45. A method according to claim 44, wherein said lactic acid bacteria are selected from the group consisting of *Lactobacillus plantarum, Lactobacillus brevis, Lactobacillus acidophilus, Lactobacillus reuteri, Lactococcus lactis, Weissella confusa* and *Bifidobacterium longum.*

46. A method according to any of claims 25 to 45, wherein said instant active dry yeasts are baker's yeasts, including sugar- and freeze tolerant yeasts, oenological yeasts, brewery yeasts, ethanol producing yeasts, and/or probiotic yeasts.

47. A method according to claim 46, wherein said yeasts are Saccharomyces yeast strains.
